# EUROPÄISCHE PATENTANMELDUNG

(11) **EP 1 132 476 A1**
(43) Veröffentlichungstag der Anmeldung: **12.09.2001**
(21) Anmeldenummer: 01103590.4
(22) Anmeldetag: 21.02.2001
(51) Int. Cl.: C12N 15/55, C12N 9/14, C12N 1/21, C07K 16/40, C12Q 1/68, G01N 33/50, G01N 33/68, A01K 67/033

(54) **Anorganische Pyrophosphatasen aus Lepidopteren**

(30) Priorität: 06.03.2000 DE 10010938
(71) Anmelder: BAYER AG, 51368 Leverkusen (DE)
(72) Erfinder: Raming, Klaus, Dr., Redwood City, CA 94061 (US)

(57) **Zusammenfassung**

Die Erfindung betrifft Polypeptide, welche die biologische Aktivität von anorganischer Pyrophosphatase ausüben, sowie Nukleinsäuren, die für diese Polypeptide codieren und insbesondere deren Verwendung zum Auffinden von Wirkstoffen für den Pflanzenschutz und für die Tiergesundheit.

SEQUENZPROTOKOLL

<110> Bayer Aktiengesellschaft

<120> Anorganische Pyrophosphatasen aus Lepidopteren

<130> Le A 34 331

<140>
<141>

<150> DE 100 10 938.1
<151> 2000-03-06

<160> 2

<170> PatentIn Ver. 2.1

<210> 1
<211> 867
<212> DNA
<213> Heliothis virescens

<220>
<221> CDS
<222> (1)..(864)

<400> 1
atg tac atc gcg gaa gaa cga ggc tcg gct tac gcg cct gat tac cgg    48
Met Tyr Ile Ala Glu Glu Arg Gly Ser Ala Tyr Ala Pro Asp Tyr Arg
  1               5                  10                  15

gtg ttc ttc aag gat gag agc ggg ccg atc tcg ccg ctg cat gac atc    96
Val Phe Phe Lys Asp Glu Ser Gly Pro Ile Ser Pro Leu His Asp Ile
             20                  25                  30

ccg ctg tgg gcc gac aag agc aag cgg ctc gtc aac atg gtg gtg gag    144
Pro Leu Trp Ala Asp Lys Ser Lys Arg Leu Val Asn Met Val Val Glu
         35                  40                  45

gtg ccg cgc tgg acc aat gcc aag atg gag atc agc ctc ggc gag ccg    192
Val Pro Arg Trp Thr Asn Ala Lys Met Glu Ile Ser Leu Gly Glu Pro
     50                  55                  60

ctc aac ccc atc aag cag gac gtg aag aag ggg gcg ctg cgc ttc gtc    240
Leu Asn Pro Ile Lys Gln Asp Val Lys Lys Gly Ala Leu Arg Phe Val
 65                  70                  75                  80

agc aac gtg ttc ccg cac cgc ggc tac atc tgg aac tac ggc gcg ctg    288
Ser Asn Val Phe Pro His Arg Gly Tyr Ile Trp Asn Tyr Gly Ala Leu
                 85                  90                  95

ccg cag acg tgg gag aac ccg cac cac gtg gac ccg ggc acg cag gcg    336
Pro Gln Thr Trp Glu Asn Pro His His Val Asp Pro Gly Thr Gln Ala
            100                 105                 110

cgc ggg gac aac gac ccc atc gac gtg atc gag atc ggc gag cgc gtg    384
Arg Gly Asp Asn Asp Pro Ile Asp Val Ile Glu Ile Gly Glu Arg Val
        115                 120                 125

gcg gcg cgc ggc gac gtg tac ccc gtg cgc atc ctc ggc acg ctg gcg    432
Ala Ala Arg Gly Asp Val Tyr Pro Val Arg Ile Leu Gly Thr Leu Ala
    130                 135                 140

ctc atc gac gag ggc gag acc gac tgg aag ctc atc gcc atc gac gcg    480
Leu Ile Asp Glu Gly Glu Thr Asp Trp Lys Leu Ile Ala Ile Asp Ala
145                 150                 155                 160

cgc gac ccc agc gcc gcc aag ctg cac gac gtg gcc gac gtg gag acg    528
Arg Asp Pro Ser Ala Ala Lys Leu His Asp Val Ala Asp Val Glu Thr
                165                 170                 175

cac ttc ccc ggc ctg ctg cgc gcc acc gtc gag tgg ttc cgc ctc tac    576
His Phe Pro Gly Leu Leu Arg Ala Thr Val Glu Trp Phe Arg Leu Tyr
            180                 185                 190

aag gtg ccg gac ggc aag ccc gtc aac cgc ttc gcc ttc gac ggc gag    624
Lys Val Pro Asp Gly Lys Pro Val Asn Arg Phe Ala Phe Asp Gly Glu
        195                 200                 205

gcc aag aac gcc gag ttc gcc tac aag gtc gtg gac gag gtg cac gag    672
Ala Lys Asn Ala Glu Phe Ala Tyr Lys Val Val Asp Glu Val His Glu
    210                 215                 220

ttc tgg cgc tcg ctg gtg gcg ggc gac gtg gcg gac gcg acg gac atc    720
Phe Trp Arg Ser Leu Val Ala Gly Asp Val Ala Asp Ala Thr Asp Ile
225                 230                 235                 240

agc aag acc aac gtg agc gtg gag ggc agc gtg ggg cgg gtg gag cgc    768
Ser Lys Thr Asn Val Ser Val Glu Gly Ser Val Gly Arg Val Glu Arg
                245                 250                 255

gcg gac gcg gcg gcg gtg ctg gcg aag gcg ccg ccg cgt gga ctg ccg    816
Ala Asp Ala Ala Ala Val Leu Ala Lys Ala Pro Pro Arg Gly Leu Pro
            260                 265                 270

cag ccc atc cca cag tcc gtg gac aag tgg cac ttc ctc tcc agc ctg    864
Gln Pro Ile Pro Gln Ser Val Asp Lys Trp His Phe Leu Ser Ser Leu
        275                 280                 285

tga                                                                867


<210> 2
<211> 288
<212> PRT
<213> Heliothis virescens

<400> 2
Met Tyr Ile Ala Glu Glu Arg Gly Ser Ala Tyr Ala Pro Asp Tyr Arg
  1               5                  10                  15

Val Phe Phe Lys Asp Glu Ser Gly Pro Ile Ser Pro Leu His Asp Ile
             20                  25                  30

Pro Leu Trp Ala Asp Lys Ser Lys Arg Leu Val Asn Met Val Val Glu
         35                  40                  45

Val Pro Arg Trp Thr Asn Ala Lys Met Glu Ile Ser Leu Gly Glu Pro
     50                  55                  60

Leu Asn Pro Ile Lys Gln Asp Val Lys Lys Gly Ala Leu Arg Phe Val
 65                  70                  75                  80

Ser Asn Val Phe Pro His Arg Gly Tyr Ile Trp Asn Tyr Gly Ala Leu
                 85                  90                  95

Pro Gln Thr Trp Glu Asn Pro His His Val Asp Pro Gly Thr Gln Ala
            100                 105                 110

Arg Gly Asp Asn Asp Pro Ile Asp Val Ile Glu Ile Gly Glu Arg Val
        115                 120                 125

Ala Ala Arg Gly Asp Val Tyr Pro Val Arg Ile Leu Gly Thr Leu Ala
    130                 135                 140

Leu Ile Asp Glu Gly Glu Thr Asp Trp Lys Leu Ile Ala Ile Asp Ala
145                 150                 155                 160

Arg Asp Pro Ser Ala Ala Lys Leu His Asp Val Ala Asp Val Glu Thr
                165                 170                 175

His Phe Pro Gly Leu Leu Arg Ala Thr Val Glu Trp Phe Arg Leu Tyr
            180                 185                 190

Lys Val Pro Asp Gly Lys Pro Val Asn Arg Phe Ala Phe Asp Gly Glu
        195                 200                 205

Ala Lys Asn Ala Glu Phe Ala Tyr Lys Val Val Asp Glu Val His Glu
    210                 215                 220

Phe Trp Arg Ser Leu Val Ala Gly Asp Val Ala Asp Ala Thr Asp Ile
225                 230                 235                 240

Ser Lys Thr Asn Val Ser Val Glu Gly Ser Val Gly Arg Val Glu Arg
                245                 250                 255

Ala Asp Ala Ala Ala Val Leu Ala Lys Ala Pro Pro Arg Gly Leu Pro
            260                 265                 270

Gln Pro Ile Pro Gln Ser Val Asp Lys Trp His Phe Leu Ser Ser Leu
        275                 280                 285

## Beschreibung

Die Erfindung betrifft Polypeptide, welche die biologische Aktivität von anorganischer Pyrophosphatase ausüben, sowie Nukleinsäuren, die für diese Polypeptide codieren und insbesondere deren Verwendung zum Auffinden von Wirkstoffen für den Pflanzenschutz und für die Tiergesundheit.

Die anorganische Pyrophosphatase ist ein essentielles Enzym, welches für die Kontrolle des zellulären Spiegels von anorganischem Pyrophosphat wichtig ist. Das Enzym katalysiert die Hydrolyse von anorganischem Pyrophosphat, welches in vielen biosynthetischen Reaktionen in der Zelle gebildet wird, in anorganisches Phosphat. Anorganisches Pyrophosphat wird in der Zelle bei verschiedenen metabolischen Prozessen gebildet. Es wird z.B. sowohl bei der Synthese von Biopolymeren wie DNA, RNA, Proteinen und Polysacchariden gebildet als auch bei der Synthese von cAMP oder cGMP. Ohne die anorganische Pyrophosphatase käme es in der Zelle zu einer Anreicherung von anorganischem Pyrophosphat, welches für die Zelle toxisch ist, da die oben genannten Synthesen in Gegenwart von hohen Konzentrationen von anorganischem Pyrophoshat reversibel sind. Daher ist dieses Enzym für alle pro-und eukaryotische Zellen essentiell, was in verschiedenen Studien bei Bakterien, Hefen und Insekten belegt ist (Chen J. et al. 1990, Lundin M. et al. 1991, Spradling et al. 1999).

Anorganische Pyrophosphatasen sind vor allem in Bakterien und Hefen gut untersucht. In Insekten ist bislang nur die Nukleotidsequenz bzw. Aminosäuresequenz von Drosophila melanogaster bekannt (Gdula D. A. et al. 1998).

Es ist daher von großer praktischer Bedeutung, beispielsweise für die Suche nach neuen Insektiziden für Schadinsekten, wie Heliothis virescens, anorganische Pyrophosphatase insbesondere aus Lepidopteren bereitzustellen.

Der vorliegenden Erfindung liegt somit die Aufgabe zugrunde, anorganische Pyrophosphatase insbesondere aus Lepidopteren, wie Heliothis virencens, und darauf aufbauende Testsysteme mit einem hohen Durchsatz an Testverbindungen (High Throughput Screening Assays; HTS-Assays) bereitzustellen.

Die Aufgabe wird gelöst durch die Bereitstellung von Polypeptiden, welche zumindest eine biologische Aktivität von anorganischen Pyrophosphatasen ausüben und eine Aminosäuresequenz umfassen, die eine zumindest 70 %ige Identität, vorzugsweise eine zumindest 80 %ige Identität, besonders bevorzugt eine zumindest 90 %ige Identität, ganz besonders bevorzugt eine zumindest 95 %ige Identität, mit einer Sequenz gemäß SEQ ID NO: 2 über eine Länge von wenigstens 20, vorzugsweise wenigstens 25, besonders bevorzugt wenigstens 30 fortlaufenden Aminosäuren und ganz besonders bevorzugt über deren Gesamtlänge aufweisen.

Der Grad der Identität der Aminosäuresequenzen wird vorzugsweise bestimmt mit Hilfe des Programms GAP aus dem Programmpaket GCG, Version 9.1 unter Standardeinstellungen (Devereux et al. 1984).

Der Ausdruck "Polypeptide", wie er hierin verwendet wird, bezieht sich sowohl auf kurze Aminosäureketten, die gewöhnlich als Peptide, Oligopeptide oder Oligomere bezeichnet werden, als auch auf längere Aminosäureketten, die gewöhnlich als Proteine bezeichnet werden. Er umfasst Aminosäureketten, die entweder durch natürliche Prozesse, wie posttranslationale Prozessierung, oder durch chemische Verfahren, die Stand der Technik sind, modifiziert sein können. Solche Modifikationen können an verschiedenen Stellen und mehrfach in einem Polypeptid vorkommen, wie beispielsweise an dem Peptid-Rückgrat, an der Aminosäure-Seitenkette, am Amino- und/oder am Carboxy-Terminus. Sie umfassen beispielsweise Acetylierungen, Acylierungen, ADP-Ribosylierungen, Amidierungen, kovalente Verknüpfungen mit Flavinen, Häm-Anteilen, Nukleotiden oder Nukleotid-Derivaten, Lipiden oder Lipid-Derivaten oder Phophatidylinositol, Cyclisierungen, Disulfidbrückenbildungen, Demethylierungen, Cystin-Bildungen, Formylierungen, gamma-Carboxylierungen, Glycosylierungen, Hydroxylierungen, Iodierungen, Methylierungen, Myristoylierungen, Oxidationen, proteolytische Prozessierungen, Phosphorylierungen, Selenoylierungen und tRNA-vermittelte Additionen von Aminosäuren.

Die erfindungsgemäßen Polypeptide können in der Form "reifer" Proteine oder als Teile größerer Proteine, z.B. als Fusionsproteine, vorliegen. Weiterhin können sie Sezernierungs- oder "Leader"-Sequenzen, Pro-Sequenzen, Sequenzen, die eine einfache Reinigung ermöglichen, wie mehrfache Histidin-Reste, oder zusätzliche stabilisierende Aminosäuren aufweisen.

Die erfindungsgemäßen Polypeptide müssen nicht vollständige Enzyme darstellen, sondern können auch nur Fragmente davon sein, solange sie zumindest noch eine biologische Aktivität der vollständigen Enzyme aufweisen. Polypeptide, die im Vergleich zu einer anorganischen Pyrophosphatase mit einer Aminosäuresequenz gemäß SEQ ID NO: 2 eine um 50 % höhere oder verminderte Aktivität ausüben, werden noch als erfindungsgemäß betrachtet. Dabei müssen die erfindungsgemäßen Polypeptide nicht von der anorganischen Pyrophosphatase aus Heliothis virescens ableitbar sein. Als erfindungsgemäß werden auch Polypeptide betrachtet, die anorganischen Pyrophosphatasen anderer Lepidopteren als Heliothis virescens entsprechen oder Fragmente davon, die noch die biologische Aktivität einer anorganischen Pyrophosphatase ausüben können.

Die erfindungsgemäßen Polypeptide können im Vergleich zu der entsprechenden Region natürlich vorkommender anorganischer Pyrophosphatasen Deletionen oder Aminosäuresubstitutionen aufweisen, solange sie zumindest noch eine biologische Aktivität der vollständigen Enzyme ausüben. Konservative Substitutionen sind bevorzugt. Solche konservativen Substitutionen umfassen Variationen, wobei eine Aminosäure durch eine andere Aminosäure aus der folgenden Gruppe ersetzt wird:
1. Kleine aliphatische, nicht-polare oder wenig polare Reste: Ala, Ser, Thr, Pro und Gly;
2. Polare, negativ geladene Reste und deren Amide: Asp, Asn, Glu und Gln;
3. Polare, positiv geladene Reste: His, Arg und Lys;
4. Große aliphatische, nicht-polare Reste: Met, Leu, Ile, Val und Cys; und
5. Aromatische Reste: Phe, Tyr und Trp.

### Die folgende Liste zeigt bevorzugte konservative Substitutionen:

| **Ursprünglicher Rest** | **Substitution** |
|---|---|
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Der Ausdruck "biologische Aktivität einer anorganischen Pyrophosphatase", wie er hierin verwendet wird, bedeutet Hydrolyse von anorganischem Pyrophosphat.

Eine bevorzugte Ausführungsform der erfindungsgemäßen Polypeptide stellt die anorganische Pyrophosphatase von Heliothis virescens dar, welche die Aminosäuresequenz gemäß SEQ ID NO: 2 besitzt.

Gegenstand der vorliegenden Erfindung sind auch Nukleinsäuren, die für die erfindungsgemäßen Polypeptide codieren.

Bei den erfindungsgemäßen Nukleinsäuren handelt es sich insbesondere um einzelsträngige oder doppelsträngige Desoxyribonukleinsäuren (DNA) oder Ribonukleinsäuren (RNA). Bevorzugte Ausführungsformen sind Fragmente genomischer DNA, die Introns enthalten können, und cDNAs.

Eine bevorzugte Ausführungsform der erfindungsgemäßen Nukleinsäuren stellt eine cDNA dar, welche die Nukleinsäuresequenz gemäß SEQ ID NO:1 aufweist.

Nukleinsäuren, welche unter stringenten Bedingungen an die Sequenzen gemäß SEQ ID NO: 1 hybridisieren, sind ebenfalls von der vorliegenden Erfindung umfasst.

Der Ausdruck "hybridisieren", wie er hierin verwendet wird, beschreibt den Vorgang, bei welchem ein einzelsträngiges Nukleinsäuremolekül mit einem komplementären Strang eine Basenpaarung eingeht. Auf diese Weise können ausgehend von der hierin offenbarten Sequenzinformation beispielsweise DNA-Fragmente aus anderen Lepidopteren als Heliothis virescens isoliert werden, welche für Polypeptide mit der biologischen Aktivität von anorganischer Pyrophosphatase codieren.

Bevorzugte Hybridisierungsbedingungen sind nachstehend angegeben:
Hybridisierungslösung: 6X SSC / 0 % Formamid, bevorzugte Hybridisierungslösung: 6X SSC / 25 % Formamid
Hybridisierungstemperatur: 34°C, bevorzugte Hybridisierungstemperatur: 42°C
1. Waschschritt: 2X SSC bei 40°C,
2. Waschschritt: 2X SSC bei 45°C; bevorzugter 2. Waschschritt: 0,6X SSC bei 55°C; besonders bevorzugter 2. Waschschritt: 0,3X SSC bei 65°C.

Weiterhin sind von der vorliegenden Erfindung Nukleinsäuren umfasst, die eine zumindest 70 %ige Identität, vorzugsweise eine zumindest 80 %ige Identität, besonders bevorzugt eine zumindest 90 %ige Identität, ganz besonders bevorzugt eine zumindest 95 %ige Identität, mit einer Sequenz gemäß SEQ ID NO: 1 über eine Länge von wenigstens 20, vorzugsweise wenigstens 25, besonders bevorzugt wenigstens 30 fortlaufenden Nukleotiden und ganz besonders bevorzugt über deren Gesamtlänge aufweisen.

Der Grad der Identität der Nukleinsäuresequenzen wird vorzugsweise bestimmt mit Hilfe des Programms GAP aus dem Programmpaket GCG, Version 9.1 unter Standardeinstellungen.

Gegenstand der vorliegenden Erfindung sind weiterhin DNA-Konstrukte, die eine erfindungsgemäße Nukleinsäure und einen heterologen Promotor umfassen.

Der Ausdruck "heterologer Promotor", wie er hierin verwendet wird, bezieht sich auf einen Promotor, der andere Eigenschaften als derjenige Promotor aufweist, der im Ursprungsorganismus die Expression des betreffenden Gens kontrolliert. Der Ausdruck "Promotor", wie er hierin verwendet wird, bezieht sich allgemein auf Expressionskontrollsequenzen.

Die Auswahl von heterologen Promotoren ist davon abhängig, ob zur Expression pro- oder eukaryotische Zellen oder zellfreie Systeme verwendet werden. Beispiele für heterologe Promotoren sind der frühe oder späte Promotor des SV40, des Adenovirus oder des Cytomegalovirus, das lac-System, das trp-System, die Haupt-Operator- und Promotorregionen des Phagen lambda, die Kontrollregionen des fd-Hüllproteins, der Promotor der 3-Phosphoglyceratkinase, der Promotor der Sauren Phosphatase und der Promotor des α-Mating-Faktors der Hefe.

Gegenstand der Erfindung sind weiterhin Vektoren, die eine erfindungsgemäße Nukleinsäure bzw. ein erfindungsgemäßes DNA-Konstrukt enthalten. Als Vektoren können alle in molekularbiologischen Laboratorien verwendete Plasmide, Phasmide, Cosmide, YACs oder künstliche Chromosomen verwendet werden.

Gegenstand der vorliegenden Erfindung sind auch Wirtszellen, die eine erfindungsgemäße Nukleinsäure, ein erfindungsgemäßes DNA-Konstrukt oder einen erfindungsgemäßen Vektor enthalten.

Der Ausdruck "Wirtszelle", wie er hierin verwendet wird, bezieht sich auf Zellen, die natürlicherweise die erfindungsgemäßen Nukleinsäuren nicht enthalten.

Als Wirtszellen eignen sich sowohl prokaryotische Zellen, wie Bakterien der Gattungen Bacillus, Pseudomonas, Streptomyces, Streptococcus, Staphylococcus, vorzugsweise E. coli, als auch eukaryotische Zellen, wie Hefen, Säuger-, Amphibien-, Insekten- oder Pflanzenzellen. Bevorzugte eukaryotische Wirtszellen sind HEK-293-, Schneider S2-, Spodoptera Sf9-, Kc-, CHO-, COS1-, COS7-, HeLa-, C127-, 3T3- oder BHK-Zellen und Xenopus-Oocyten.

Weiterhin sind Antikörper Gegenstand der Erfindung, die spezifisch an die vorstehend genannten Polypeptide binden. Die Herstellung solcher Antikörper erfolgt auf die übliche Weise. Beispielsweise können solche Antikörper produziert werden durch die Injektion eines substantiell immunkompetenten Wirts mit einer für die Antikörperproduktion effektiven Menge eines erfindungsgemäßen Polypeptids oder eines Fragments davon und durch nachfolgende Gewinnung dieses Antikörpers. Weiterhin läßt sich in an sich bekannter Weise eine immortalisierte Zelllinie erhalten, die monoklonale Antikörper produziert. Die Antikörper können gegebenenfalls mit einem Nachweisreagenz markiert sein. Bevorzugte Beispiele für ein solches Nachweis-Reagenz sind Enzyme, radioaktiv markierte Elemente, fluoreszierende Chemikalien oder Biotin. Anstelle des vollständigen Antikörpers können auch Fragmente eingesetzt werden, die die gewünschten spezifischen Bindungseigenschaften besitzen. Daher erstreckt sich der Ausdruck "Antikörper", wie er hierin verwendet wird, auch auf Teile vollständiger Antikörper, wie Fa-, F(ab')₂- oder Fv-Fragmente, welche noch die Fähigkeit besitzen, an die Epitope der erfindungsgemäßen Polypeptide zu binden.

Die erfindungsgemäßen Nukleinsäuren können insbesondere zur Herstellung transgener Invertebraten verwendet werden. Diese können in Testsysteme eingesetzt werden, die auf einer vom Wildtyp abweichenden Expression der erfindungsgemäßen Polypeptide basieren. Ferner kann man auf der Grundlage der hierin offenbarten Informationen transgene Invertebraten herstellen, bei denen durch die Modifikation anderer Gene oder Promotoren eine Veränderung der Expression der erfindungsgemäßen Polypeptide eintritt.

Die Herstellung der transgenen Invertebraten erfolgt beispielsweise bei Drosophila melanogaster durch P-Element vermittelten Gentransfer (Hay et al. 1997) oder in Caenorhabditis elegans durch Transposon-vermittelten Gentransfer (z.B. durch Tcl; Plasterk 1996).

Gegenstand der Erfindung sind somit auch transgene Invertebraten, die zumindest eine der erfindungsgemäßen Nukleinsäuren enthalten, vorzugsweise transgene Invertebraten der Arten Drosophila melanogaster oder Caenorhabditis elegans, sowie deren transgene Nachkommen. Vorzugsweise enthalten die transgenen Invertebraten die erfindungsgemäßen Polypeptide in einer vom Wildtyp abweichenden Form .

Gegenstand der vorliegenden Erfindung sind weiterhin Verfahren zum Herstellen der erfindungsgemäßen Polypeptide. Zur Herstellung der Polypeptide, die von den erfindungsgemäßen Nukleinsäuren codiert werden, können Wirtszellen, die eine der erfindungsgemäßen Nukleinsäuren enthalten, unter geeigneten Bedingungen kultiviert werden. Dabei kann die zu exprimierende Nukleinsäure an die "Codon Usage" der Wirtszellen angepaßt werden. Die gewünschten Polypeptide können danach auf übliche Weise aus den Zellen oder dem Kulturmedium isoliert werden. Die Polypeptide können auch in *in vitro-*Systemen hergestellt werden.

Ein schnelles Verfahren zum Isolieren der erfindungsgemäßen Polypeptide, die von Wirtszellen unter Verwendung einer erfindungsgemäßen Nukleinsäure synthetisiert werden, beginnt mit der Expression eines Fusionsproteins, wobei der Fusionspartner auf einfache Weise affinitätsgereinigt werden kann. Der Fusionspartner kann beispielsweise Glutathion S-Transferase sein. Das Fusionsprotein kann dann an einer Glutathion-Affinitätssäule gereinigt werden. Der Fusionspartner kann durch partielle proteolytische Spaltung beispielsweise an Linkem zwischen dem Fusionspartner und dem zu reinigenden erfindungsgemäßen Polypeptid abgetrennt werden. Der Linker kann so gestaltet werden, dass er Ziel-Aminosäuren, wie Arginin- und Lysin-Reste einschließt, die Stellen für eine Spaltung durch Trypsin definieren. Um solche Linker zu erzeugen, können Standard-Klonierungsverfahren unter Verwendung von Oligonukleotiden angewendet werden.

Weitere mögliche Reinigungsverfahren basieren auf präparativer Elektrophorese, FPLC, HPLC (z.B. unter Anwendung von Gelfiltrations-, Reversphasen- oder leicht hydrophoben Säulen), Gelfiltration, differentielle Präzipitation, Ionenaustausch-Chromatographie und Affinitätschromatographie.

Die Ausdrücke "Isolierung oder Reinigung", wie sie hierin verwendet werden, bedeuten, dass die erfindungsgemäßen Polypeptide von anderen Proteinen oder anderen Makromolekülen der Zelle oder des Gewebes abgetrennt werden. Vorzugsweise ist eine die erfindungsgemäßen Polypeptide enthaltende Zusammensetzung hinsichtlich des Proteingehalts gegenüber einer Präparation aus den Wirtszellen mindestens 10-fach und besonders bevorzugt mindestens 100-fach angereichert.

Die erfindungsgemäßen Polypeptide können auch ohne Fusionspartner mit Hilfe von Antikörpern, die an die Polypeptide binden, affinitätsgereinigt werden.

Ferner sind auch Verfahren zum Herstellen der erfindungsgemäßen Nukleinsäuren Gegenstand der vorliegenden Erfindung. Die erfindungsgemäßen Nukleinsäuren können auf die übliche Weise hergestellt werden. Beispielsweise können die Nukleinsäuremoleküle vollständig chemisch synthetisiert werden. Man kann auch nur kurze Stücke der erfindungsgemäßen Sequenzen chemisch synthetisieren und solche Oligonukleotide radioaktiv oder mit einem Fluoreszenzfarbstoff markieren. Die markierten Oligonukleotide können verwendet werden, um ausgehend von Insekten-mRNA hergestellte cDNA-Banken oder ausgehend von genomischer Insekten-DNA hergestellte genomische Banken zu durchsuchen. Klone, an die die markierten Oligonukleotide hybridisieren, werden zur Isolierung der betreffenden DNA ausgewählt. Nach der Charakterisierung der isolierten DNA erhält man auf einfache Weise die erfindungsgemäßen Nukleinsäuren.

Die erfindungsgemäßen Nukleinsäuren können auch mittels PCR-Verfahren unter Verwendung chemisch synthetisierter Oligonukleotide hergestellt werden.

Der Ausdruck "Oligonukleotid(e)", wie er hierin verwendet wird, bedeutet DNA-Moleküle, die aus 10 bis 50 Nukleotiden, vorzugsweise 15 bis 30 Nukleotiden, bestehen. Sie werden chemisch synthetisiert und können als Sonden verwendet werden.

Mit Hilfe der erfindungsgemäßen Nukleinsäuren bzw. Polypeptiden können neue Wirkstoffe für den Pflanzenschutz bzw. pharmazeutische Wirkstoffe für die Behandlung von Mensch und Tier, wie chemische Verbindungen, welche als Modulatoren, insbesondere als Inhibitoren oder Effektoren, die Eigenschaften der erfindungsgemäßen anorganischen Pyrophosphatase verändern, identifiziert werden. Dazu wird beispielsweise ein rekombinantes DNA-Molekül, das die erfindungsgemäße Nukleinsäure umfasst, in eine geeignete Wirtszelle eingebracht. Die Wirtzelle wird unter Bedingungen kultiviert, welche die Expression eines erfindungsgemäßen Polypeptids erlauben. Eine anschließende Reiniung des Polypeptids ermöglicht dann den Einsatz in einem enzymatischen Assay Eine Veränderung der Enzymeigenschaften kann - wie nachstehend in Beispiel 2 beschrieben - detektiert werden. Auf diese Weise ist es möglich, beispielsweise insektizide Substanzen aufzufinden.

Ein Assay mit hohem Durchsatz, in dem z.B. eine inhibierende Wirkung von chemischen Substanzen auf die anorganische Pyrophosphatase detektiert werden können, ist der Malachit-Grün-Test (Lanzetta 1979).

Der Ausdruck "Effektor", wie er hierin verwendet wird, bezieht sich auf ein Molekül, das anorganische Pyrophosphatase aktiviert.

Der Ausdruck "Inhibitor", wie er hierin verwendet wird, bezieht sich auf ein Molekül, das anorganische Pyrophosphatase hemmt.

Der Ausdruck "Modulator", wie er hierin verwendet wird, stellt den Oberbegriff zu Inhibitor bzw. Effektor dar. Modulatoren können kleine organisch-chemische Moleküle, Peptide oder Antikörper sein, die an die erfindungsgemäßen Polypeptide binden. Weiterhin können Modulatoren kleine organisch-chemische Moleküle, Peptide oder Antikörper sein, die an ein Molekül binden, welches wiederum an die erfindungsgemäßen Polypeptide bindet, und dadurch deren biologische Aktivität beeinflusst. Modulatoren können Mimetika von natürlichen Substraten und Liganden darstellen.

Vorzugsweise handelt es sich bei den Modulatoren um kleine organisch-chemische Verbindungen.

Die Bindung der Modulatoren an die erfindungsgemäßen Polypeptide kann die zellulären Vorgänge auf eine Weise verändern, die zum Absterben der damit behandelten Insekten führt.

Daher erstreckt sich die vorliegende Erfindung auch auf die Verwendung von Modulatoren der erfindungsgemäßen Polypeptide als Insektizide.

Unter Verwendung von Wirtszellen oder transgenen Invertebraten, die die erfindungsgemäßen Nukleinsäuren enthalten, ist es auch möglich, Substanzen aufzufinden, welche die Expression des Enzyms verändern.

Die vorstehend beschriebenen erfindungsgemäßen Nukleinsäuren können außerdem zum Auffinden von Genen verwendet werden, die für funktionell ähnliche anorganische Pyrophosphatasen in Insekten codieren. Unter funktionell ähnlichen Enzymen werden gemäß der vorliegenden Erfindung Enzyme verstanden, welche sich zwar hinsichtlich der Aminosäuresequenz von den hierin beschriebenen Polypeptiden unterscheiden, aber im wesentlichen dieselbe biologische Aktivität ausüben.

### Erläuterungen zum Sequenzprotokoll und zu den Abbildungen:

SEQ ID NO: 1 zeigt die Nukleotid- und Aminosäuresequenz der für anorganische Pyrophosphatase codierenden cDNA. SEQ ID NO: 2 zeigt die Aminosäuresequenz einer anorganischen Pyrophosphatase aus Heliothis virescens.

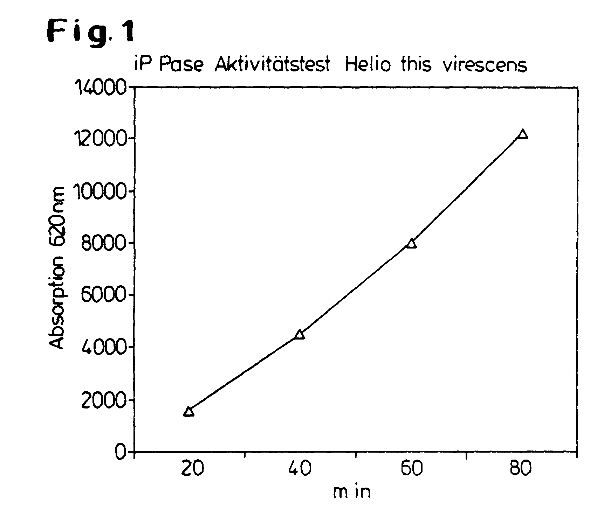

Die Abbildung 1 zeigt eine Zeitkinetik der Farbkomplexentwicklung. Anorganische Pyrophosphatase mit einer Aminosäuresequenz gemäß SEQ ID NO: 2 wurde in E. coli exprimiert und aufgereinigt. Im Assay wurden 80 fg Protein und 80 µM Substrat eingesetzt.

### Beispiele:

### Beispiel 1

Isolierung der beschriebenen Nukleinsäure-Moleküle

Die Manipulation von Polynukleotiden erfolgte nach Standardmethoden der rekombinanten DNA-Technologie (Sambrook et al. 1989). Die bioinformatische Bearbeitung von Nukleotid- und Proteinsequenzen erfolgten mit dem Programmpaket GCG Version 9.1 (GCG Genetics Computer Group, Inc., Madison Wisconsin, USA).

### Beispiel 2

### Generierung der Expressionskonstrukte

Mittels Polymerasekettenreaktion (PCR) wurden die Sequenzbereiche der SEQ ID NO: 1 amplifiziert und in den Vektor pQE60 (Qiagen, Hilden) einkloniert.

### Heterologe Expression

Die heterologe Expression der anorganischen Pyrophosphatase aus Heliothis virescens erfolgte in E. coli (M15 [pREP4]) gemäß den Angaben des Herstellers (The QIAexpressionist, 3. Auflage, März 1999, Qiagen, Hilden).

### Enzym-Aktivitätsmessung

Zur Bestimmung der Enzymaktivität wurde das Phosphat als Produkt der Reaktion von anorganischer Pyrophosphatase mit Hilfe des Malachit-Grün-Tests (Lanzetta 1979) bestimmt. Dabei wird Phosphat über eine Komplexbildung mit Malachit-Grün und anschließender fotometrischer Detektion bei 620 nm quantitativ bestimmt.

### Literatur:

Chen, J. et al. 1990, J. Bacteriol. 172, 5686-5689
Devereux et al. 1984, Nucleic Acids Research 12, 387
Gdula D. A. et al. 1998, Genes & Development 12, 3206-3216
Hay et al. 1997, Proceedings of The National Academy of Sciences of The United States of America 94 (10), 5195-5200
Lanzetta et al. 1979, Analytical Biochemistry 100, 95-97
Lundin, M. et al. 1991, J. Biol. Chem. 266, 12168-12172
Plasterk 1996, The Tc1/mariner transposon family, Transposable Elements/Current Topics in Microbiology and Immunology 204, 125-143
Sambrook et al. 1989, Molecular Cloning, A Laboratory Manual, 2nd ed. Cold Spring Harbour Press
Spradling AC, Stern D, Beaton A, Rhem EJ, Laverty T, Mozden N, Misra S, Rubin GM Genetics. 1999 Sep;153(1):135-77.

## Patentansprüche

1. Polypeptid, welches die biologische Aktivität einer anorganischen Pyrophosphatase ausübt und eine Aminosäuresequenz umfasst, die eine zumindest 70 %ige Identität mit einer Sequenz gemäß SEQ ID NO: 2 aufweist.

2. Polypeptid gemäß Anspruch 1, **dadurch gekennzeichnet, dass** die Aminosäuresequenz der Sequenz gemäß SEQ ID NO: 2 entspricht.

3. Nukleinsäure umfassend eine Nukleotidsequenz, die für ein Polypeptid gemäß Anspruch 1 oder 2 codiert.

4. Nukleinsäure gemäß Anspruch 3, **dadurch gekennzeichnet, dass** es sich um einzelsträngige oder doppelsträngige DNA oder RNA handelt.

5. Nukleinsäure gemäß Anspruch 4, **dadurch gekennzeichnet, dass** es sich um Fragmente genomischer DNA oder cDNA handelt.

6. Nukleinsäure gemäß Anspruch 3, **dadurch gekennzeichnet, dass** die Nukleotidsequenz der Sequenz gemäß SEQ ID NO: 1 entspricht.

7. Nukleinsäure gemäß Anspruch 3, **dadurch gekennzeichnet, dass** sie unter stringenten Bedingungen an die Sequenz gemäß SEQ ID NO:1 hybridisiert.

8. DNA-Konstrukt umfassend eine Nukleinsäure gemäß einem der Ansprüche 3 bis 7 und einen heterologen Promotor.

9. Vektor umfassend eine Nukleinsäure gemäß einem der Ansprüche 3 bis 7 oder ein DNA-Konstrukt gemäß Anspruch 8.

10. Vektor gemäß nach Anspruch 9, **dadurch gekennzeichnet, dass** die Nukleinsäure funktionell mit regulatorischen Sequenzen verknüpft ist, die die Expression der Nukleinsäure in pro- oder eukaryotischen Zellen gewährleisten.

11. Wirtszelle enthaltend eine Nukleinsäure gemäß einem der Ansprüche 3 bis 7, ein DNA-Konstrukt gemäß Anspruch 8 oder einen Vektor gemäß Anspruch 9 oder 10.

12. Wirtszelle gemäß Anspruch 11, **dadurch gekennzeichnet, dass** es sich um eine prokaryotische Zelle, insbesondere um E. coli, handelt.

13. Wirtszelle gemäß Anspruch 11, **dadurch gekennzeichnet, dass** es sich um eine eukaryotische Zelle, insbesondere um eine Säuger- oder Insektenzelle, handelt.

14. Antikörper, welcher spezifisch an ein Polypeptid gemäß Anspruch 1 oder 2 bindet.

15. Transgener Invertebrat enthaltend eine Nukleinsäure gemäß einem der Ansprüche 3 bis 7.

16. Transgener Invertebrat nach Anspruch 15, **dadurch gekennzeichnet, dass** es sich um Drosophila melanogaster oder Caenorhabditis elegans handelt.

17. Transgene Nachkommen eines Invertebraten gemäß Anspruch 15 oder 16.

18. Verfahren zum Herstellen eines Polypeptids gemäß Anspruch 1 oder 2, umfassend
(a) das Kultivieren einer Wirtszelle gemäß einem der Ansprüche 11 bis 13 unter Bedingungen, die die Expression der Nukleinsäure gemäß einem der Ansprüch 3 bis 7 gewährleisten, oder
(b) das Exprimieren einer Nukleinsäure gemäß einem der Ansprüche 3 bis 7 in einem *in vitro-System,* und
(c) die Gewinnung des Polypeptids aus der Zelle, dem Kulturmedium oder dem *in vitro-System.*

19. Verfahren zum Herstellen einer Nukleinsäure gemäß einem der Ansprüche 3 bis 7, umfassend die folgenden Schritte:
(a) Vollständige chemische Synthese auf an sich bekannte Weise, oder
(b) chemische Synthese von Oligonukleotiden, Markieren der Oligonukleotide, Hybridisieren der Oligonukleotide an DNA einer genomischen oder cDNA-Bank, die ausgehend von genomischer DNA bzw. mRNA aus Insektenzellen hergestellt wurde, Selektieren von positiven Klonen und Isolieren der hybridisierenden DNA aus positiven Klonen, oder
(c) chemische Synthese von Oligonukleotiden und Amplifizierung der Ziel-DNA mittels PCR.

20. Verfahren zum Herstellen eines transgenen Invertebraten gemäß Anspruch 15 oder 16, umfassend das Einbringen einer Nukleinsäure gemäß einem der Ansprüche 3 bis 7 oder eines Vektors gemäß Anspruch 9 oder 10.

21. Verfahren zum Auffinden neuer Wirkstoffe für den Pflanzenschutz oder für die Tiergesundheit, insbesondere von Verbindungen, welche die Eigenschaften von Polypeptiden gemäß Anspruch 1 oder 2 verändern, umfassend die folgenden Schritte:
(a) Bereitstellen einer Wirtszelle gemäß einem der Ansprüche 11 bis 13,
(b) Kultivieren der Wirtszelle in der Gegenwart einer chemischen Verbindung oder einem Gemisch von chemischen Verbindungen, und
(c) Detektieren veränderter Eigenschaften.

22. Verfahren zum Auffinden einer chemischen Verbindung, die an ein Polypeptid gemäß Anspruch 1 oder 2 bindet, umfassend die folgenden Schritte:
(a) Inkontaktbringen eines Polypeptids gemäß Anspruch 1 oder 2, oder einer Wirtszelle gemäß einem der Ansprüche 11 bis 13 mit einer chemischen Verbindung oder einem Gemisch von chemischen Verbindungen unter Bedingungen, die die Interaktion einer chemischen Verbindung mit dem Polypeptid erlauben, und
(b) Bestimmen der chemischen Verbindung, die spezifisch an das Polypeptid bindet.

23. Verfahren zum Auffinden einer chemischen Verbindung, die die Expression eines Polypeptids gemäß Anspruch 1 oder 2 verändert, umfassend die folgenden Schritte:
(a) Inkontaktbringen einer Wirtszelle gemäß einem der Ansprüche 11 bis 13 oder eines transgenen Invertebraten gemäß Anspruch 15 oder 16 mit einer chemischen Verbindung oder einem Gemisch von chemischen Verbindungen,
(b) Bestimmen der Konzentration des Polypeptids gemäß Anspruch 1 oder 2, und
(c) Bestimmen der chemischen Verbindung, die die Expression des Polypeptids spezifisch beeinflusst.

24. Verwendung eines Polypeptids gemäß Anspruch 1 oder 2, einer Nukleinsäure gemäß einem der Ansprüche 3 bis 7, eines Vektors gemäß Anspruch 9 oder 10, einer Wirtszelle gemäß einem der Ansprüche 11 bis 13, eines Antikörpers gemäß Anspruch 14 oder eines transgenen Invertebraten gemäß Anspruch 15 oder 16 zum Auffinden neuer Wirkstoffe für den Pflanzenschutz oder für die Tiergesundheit, oder zum Auffinden von Genen, die für funktionell ähnliche Insekten-Polypeptide codieren.

25. Verwendung eines Modulators eines Polypeptids gemäß Anspruch 1 als Pflanzenschutzmittel.
